(19) Europäisches Patentamt
European Patent Office
Office européen des brevets

(11) **EP 3 192 491 B1**

(12) **EUROPÄISCHE PATENTSCHRIFT**

(45) Veröffentlichungstag und Bekanntmachung des Hinweises auf die Patenterteilung:
**08.01.2020 Patentblatt 2020/02**

(21) Anmeldenummer: **16151443.5**

(22) Anmeldetag: **15.01.2016**

(51) Int Cl.:
*A61K 8/26* *(2006.01)*  *A61K 8/27* *(2006.01)*
*A61K 8/28* *(2006.01)*  *A61K 8/39* *(2006.01)*
*A61Q 1/02* *(2006.01)*  *A61Q 15/00* *(2006.01)*
*A61Q 17/04* *(2006.01)*  *A61K 8/73* *(2006.01)*
*A61Q 19/04* *(2006.01)*  *A61Q 19/08* *(2006.01)*

(54) **ZUSAMMENSETZUNG ENTHALTEND POLYGLYCERINESTER UND HYDROXY-ALKYLMODIFIZIERTES GUAR**

COMPOSITION COMPRISING POLYGLYCEROL ESTERS AND HYDROXY-ALKYL MODIFIED GUAR

COMPOSITION CONTENANT DE L'ESTER DE POLYGLYCERINE ET DE LA GOMME DE GUAR MODIFIEE PAR UN GROUPE HYDROXY-ALKYLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**19.07.2017 Patentblatt 2017/29**

(73) Patentinhaber: **Evonik Operations GmbH**
**45128 Essen (DE)**

(72) Erfinder:
• **FRIEDRICH, Achim**
**45529 Hattingen (DE)**
• **MEYER, Jürgen**
**45134 Essen (DE)**
• **UNGER, Frank**
**46147 Oberhausen (DE)**
• **DAHL, Verena**
**51515 Kürten (DE)**
• **VON HOF, Jan Marian**
**45138 Essen (DE)**
• **SPRINGER, Oliver**
**46485 Wessel (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
EP-A1- 0 051 148    EP-A2- 1 051 966
EP-A2- 2 705 832    WO-A1-2015/132053
US-A1- 2014 301 963

Bemerkungen:
Die Akte enthält technische Angaben, die nach dem Eingang der Anmeldung eingereicht wurden und die nicht in dieser Patentschrift enthalten sind.

**Beschreibung**

Gebiet der Erfindung

Gegenstand der Erfindung sind Zusammensetzungen enthaltend Polyglycerinester und langkettig hydroxy-alkylmodifiziertes Guar.

Stand der Technik

WO2013092186 offenbart eine schweißhemmende Zusammensetzung in Form einer Öl-in-Wasser-Emulsion, die keine Mikroemulsion ist, enthaltend

a) mindestens ein schweißhemmendes Aluminiumsalz in einer Gesamtmenge von 2 - 40 Gew%, wobei sich die Gew.-%-Angaben auf das Gesamtgewicht der kristallwasserfreien und ligandenfreien Aktivsubstanz (USP) in dem Mittel beziehen, und zusätzlich dazu
b) in einer Gesamtmenge von 0, 1 - 2 Gew.-% mindestens eine oberflächenaktive Verbindung mit einem HLB-Wert im Bereich von 9 bis 15, ausgewählt aus den Partialestern eines Polyglycerins, das 3, 4 oder 5 Glycerin-Einheiten umfasst, mit einer linearen oder verzweigten, gesättigten oder ungesättigten Carbonsäure mit 8 bis 22 C-Atomen und mit einer organischen Genusssäure,
und zusätzlich dazu
c) in einer Gesamtmenge von 0,1 - 2,0 Gew.-% mindestens ein bestimmtes N-Acyl-L-glutaminsäure-Natriumsalz.

WO2015132053 offenbart Deodorant Formulierung enthaltend Ester eines Polyglycerins mit Stearinsäure, Palmitinsäure und Caprylsäure, sowie Hydroxyethyl Cellulose. Aufgabe der Erfindung war es, Zusammensetzungen bereitzustellen, die eine niedrigviskose und stabile Formulierung von emulsionsbelastenden Inhaltsstoffen, wie beispielsweise AP/Deo-Wirkstoffen, ermöglichen.

Beschreibung der Erfindung

Überraschenderweise wurde gefunden, dass die im Folgenden beschriebenen Zusammensetzungen die der Erfindung gestellte Aufgabe zu lösen vermögen.

Gegenstand der vorliegenden Erfindung sind daher Zusammensetzungen enthaltend bestimmte Polyglycerinester und hydroxy-alkylmodifiziertes Guar.

Ein Vorteil der vorliegenden Erfindung besteht darin, dass der in den erfindungsgemäßen Zusammensetzungen enthaltene Polyglycerinester vollkommen auf nachwachsenden Rohstoffen basiert.

Noch ein Vorteil der vorliegenden Erfindung ist es, dass die erfindungsgemäße Zusammensetzung sich zur Formulierung von O/W Emulsionen (Cremes, Lotionen) mit hervorragender Lagerstabilität eignet.

Ein weiterer Vorteil der vorliegenden Erfindung ist es, dass die erfindungsgemäße Zusammensetzung sich zur Formulierung PEG-freier Emulsionen, insbesondere dünnflüssiger PEG-freier Emulsionen eignet.

Ein weiterer Vorteil der vorliegenden Erfindung ist es, dass die erfindungsgemäße Zusammensetzung sich zur Formulierung PEG-freier AP/Deo oder Deo-Emulsionen, insbesondere Roll-on-Emulsionen eignet.

Ein weiterer Vorteil der vorliegenden Erfindung ist es, dass die erfindungsgemäße Zusammensetzung sich zur Formulierung von Emulsionen eignet, welche eine Fließgrenze aufweisen.

Emulsionen und Formulierungen enthaltend solchen Emulgator auf Basis der erfindungsgemäßen Zusammensetzung weisen überdies ein gutes Hautgefühl auf. Vorteilhafterweise benötigen Emulsionen und Formulierungen enthaltend die erfindungsgemäße Zusammensetzung keine parabenhaltigen Konservierungsmittel. Ein weiterer Vorteil der vorliegenden Erfindung ist es, dass die erfindungsgemäße Zusammensetzung sich zur Formulierung von Emulsionen ohne polyacrylat-basierte Verdicker eignet.

Ein weiterer Vorteil der vorliegenden Erfindung ist es, dass die erfindungsgemäße Zusammensetzung aufgrund ihrer Konsistenz gut handhabbar ist.

Ein weiterer Vorteil der vorliegenden Erfindung ist es, dass die erfindungsgemäße Zusammensetzung in Formulierungen ein leichtes Hautgefühl erzeugt.

Ein weiterer Vorteil der vorliegenden Erfindung ist es, dass die Verwendung der erfindungsgemäßen Zusammensetzung den Formulierungen feuchtigkeitsspendende Eigenschaften verleiht.

Beansprucht wird daher eine Zusammensetzung enthaltend

A) Polyglycerinester, der nach seiner vollständiger Hydrolyse

a) mindestens eine Carbonsäure mit 6 bis 14, bevorzugt 8 bis 12, Kohlenstoffatomen
b) mindestens eine Carbonsäure mit 16 bis 20, bevorzugt 18 bis 20, Kohlenstoffatomen
c) mindestens eine Carbonsäure mit 22 bis 28, bevorzugt 22 bis 24, Kohlenstoffatomen freisetzt und
der nach seiner vollständiger Hydrolyse ein Polyglycerin freisetzt, das einen mittleren Polymerisationsgrad von 3,0 bis 5,0, bevorzugt von 3,3 bis 4,7, besonders bevorzugt von 3,6 bis 4,5, aufweist,
und

B) mindestens ein hydroxy-alkylmodifziertes Guar,
dadurch gekennzeichnet, dass das hydroxy-alkylmodifizierte Guar mit HydroxyAlkylgruppen mit 12 bis 26, bevorzugt 16 bis 24, besonders bevorzugt 18 bis 22, Kohlenstoffatomen modifiziert ist.

Unter dem Begriff "Polyglycerin" im Sinne der vorliegenden Erfindung ist ein Polyglycerin zu verstehen, welches Glycerin enthält. Somit ist zur Berechnung von Mengen, Massen und dergleichen der Glycerinanteil mit zu berücksichtigen. Polyglycerine im Sinne der vorliegenden Erfindung sind somit Mischungen von Glycerin mit mindestens einem Glycerin-Oligomer. Unter Glycerin-Oligomeren sind jeweils alle entsprechenden Strukturen, z.B. also lineare und zyklische Verbindungen zu verstehen.

Analoges gilt für den Begriff "Polyglycerinester" im Zusammenhang mit der vorliegenden Erfindung.

Das angegebene Zahlenmittel der Säurereste bezieht sich bei mehr als einer der Carbonsäure a), b) oder c) jeweils auf die akkumulierte Summe aller Carbonsäuren a), b) oder c).

Der mittlere Polymerisationsgrad des Polyglycerins $N$ wird über dessen Hydroxylzahl OHV, in mg KOH/g) gemäß folgender Formel berechnet:

$$N = \frac{(112200 - 18 \cdot OHV)}{(74 \cdot OHV - 56100)}$$

Geeignete Bestimmungsmethoden zur Ermittlung der Hydroxylzahl sind insbesondere solche gemäß DGF C-V 17 a (53), Ph. Eur. 2.5.3 Method A und DIN 53240.

Alle angegebenen Prozent (%) sind, wenn nicht anders angegeben, Massenprozent.

Es werden erfindungsgemäß Zusammensetzungen bevorzugt, bei denen das nachvollständiger Hydrolyse des Polyglycerinesters freigesetzte Polyglycerin ein Massenverhältnis von Glycerin zu Diglycerin von größer 1, bevorzugt größer 1,2, besonders bevorzugt größer 1,4 aufweist.

Es werden erfindungsgemäß Zusammensetzungen besonders bevorzugt, bei denen das nach vollständiger Hydrolyse des Polyglycerinesters freigesetzte Polyglycerin 5 Gew.-% bis 30 Gew.-%, bevorzugt 7 Gew.-% bis 25 Gew.-%, besonders bevorzugt

10 Gew.-% bis 22 Gew.-%, mono-Glycerin,
1 Gew.-% bis 25 Gew.-%, bevorzugt 3 Gew.-% bis 18 Gew.-%, besonders bevorzugt 5 Gew.-% bis 15 Gew.-%, Diglycerine,
1 Gew.-% bis 25 Gew.-%, bevorzugt 1 Gew.-% bis 20 Gew.-%, besonders bevorzugt 3 Gew.-% bis 17 Gew.-%, Triglycerine und
1 Gew.-% bis 20 Gew.-%, bevorzugt 2 Gew.-% bis 15 Gew.-%, besonders bevorzugt 4 Gew.-% bis 10 Gew.-%, Tetraglycerine
enthält, wobei sich die Gewichtsprozente auf das gesamte Polyglycerin beziehen.

In diesem Zusammenhang weist das freigesetzte Polyglycerin bevorzugt
≥70 Gew.-%, bevorzugt ≥75 Gew.-%, besonders bevorzugt ≥80 Gew.-%, Polyglycerine mit einem Polymerisationsgrad von ≥2,
≥60 Gew.-%, bevorzugt ≥65 Gew.-%, besonders bevorzugt ≥70 Gew.-%, Polyglycerine mit einem Polymerisationsgrad von ≥3,
≥50 Gew.-%, bevorzugt ≥55 Gew.-%, besonders bevorzugt ≥60 Gew.-%, Polyglycerine mit einem Polymerisationsgrad von ≥4 und
≥40 Gew.-%, bevorzugt ≥45 Gew.-%, besonders bevorzugt ≥50 Gew.-%, Polyglycerine mit einem Polymerisationsgrad von ≥5
auf, wobei sich die Gewichtsprozent auf das gesamte Polyglycerin beziehen.

Der Massenanteil von Glycerin, Diglycerin, Triglycerin, Tetraglycerin sowie der Fettsäuren lässt sich im Rahmen der vorliegenden Erfindung durch zwei GC-Methoden bestimmen; diese Methoden beinhalten die alkalische Hydrolyse des erfindungsgemäßen Polyglycerinesters, Trennung des Polyglycerins von den freigesetzten Säuren und Analyse der Fettsäuren sowie der Glycerin-Oligomere (lineare und zyklische).

Hierzu werden 0,5 g des erfindungsgemäßen Polyglycerinesters in 25 ml einer ethanolischen 0,5 M KOH Lösung unter Rückfluss für 4 Stunden gekocht. Dann werden 10 ml Wasser zugegeben und der pH mit Schwefelsäure auf pH 2-3 eingestellt. Die entstandenen Carbonsäuren werden durch dreimalige Extraktion mit jeweils einem Volumen (20 ml) Petrolether abgetrennt.

Fettsäureanalyse :

Die vereinigten Extrakte werden durch Evaporation auf ca. 1 ml eingeengt. Geeignete Bestimmungsmethoden zur Ermittlung der Fettsäureverteilung sind insbesondere solche gemäß DGF C VI 11a, DGF C-VI 10 a und GAT - Ringtest 7/99. Ein 0,5 ml Aliquot des wie oben beschriebenen erhaltenen Petrioletherextraktes wird in einem Gefäß mit 1 ml einer Mischung von Acetylchlorid: Methanol (1:4) unter Ausschluss von Luftfeuchtigkeit 30 min in der Siedehitze behandelt. Die entstehenden Fettsäuremethylester werden 2 mal mit jeweils 5 ml iso-Octan extrahiert und mittels GC analysiert. Dieses wird in einem Gaschromatograph, welcher mit einem split/splitless Injektor, einer Kapillarsäule und einem Flammenionisationdetektor ausgestattet ist, unter folgenden Bedingungen durchgeführt:

| | |
|---|---|
| Injektor: | 290 °C, Split 30 ml |
| Injektionsvolumen: | 1 $\mu$l |
| Säule: | 30 m *0,32 mm HP1 0,25 $\mu$m |
| Trägergas: | Helium, constant flow, 2 mL/min |
| Temperaturprogramm: | 80 °C - 300 °C mit 8 °C/min, dann 10 Minuten bei 300 °C konditionieren. |
| Detektor: | FID bei 320 °C |
| | Wasserstoff 35 ml/min |
| | Luft 240 ml/min |
| | Make Up Gas 12 ml/min |

Die Carbonsäuren werden als ihre Methylester nach ihrer Kohlenstoffkettenlänge getrennt und ihr Massenanteil nach einer Methode des internen Standards bestimmt. Hierzu wird das GC System durch Vermessen von Fettsäuremethylester-Mischungen der zu untersuchenden Fettsäuren mit bekannter Zusammensetzung kalibriert. Mit diesem Verfahren wird die Gesamtmasse und die Massenanteile an Carbonsäure(n) erhalten, die über die Verwendung der jeweiligen Molekulargewichte eine Bestimmung der Stoffemenge(n) erlaubt. Aus der Gesamtmasse an Carbonsäure(n) lässt sich außerdem durch Subtraktion die z.B. in 0.5 g Polyglycerinester enthaltene Masse an Polyglycerin bestimmen. Mittels des Molekulargewichts des Polyglycerins lässt sich daraus die Stoffmenge des Polyglycerins bestimmen.

| | | |
|---|---|---|
| $M_p = 74 \cdot N + 18$ | mit | $M_p$ = Molekulargewicht des Polyglycerins [g/mol] <br> N = Polymerisationsgrad des Polyglycerins (zur Bestimmung des Polymerisationsgrads siehe weiter unten). |
| $n_p = \dfrac{m_p}{M_p}$ | mit | $n_p$ = Stoffmenge des Polyglycerins [mol] in 1 g Polyglycerinester <br> $m_p$ = Masse Polyglycerin in 1 g Polyglycerinester [g] <br> $M_p$ = Molekulargewicht des Polyglycerins [g/mol] |

Zusammen lassen sich aus diesen Werten die Molverhältnisse von Polyglycerin zu Carbonsäuren bestimmen.

Analyse von Glycerin, Diglycerinen, Triglycerinen und Tetraglycerinen:
Der mit Petrolether extrahierte Rückstand wird mit Bariumhydroxid auf pH 7 bis 8 eingestellt. Das ausgefallene Bariumsulfat wird durch Zentrifugation abgetrennt.
Der Überstand wird abgenommen und der Rückstand wird dreimal mit 20 ml Ethanol extrahiert.
Die vereinigten Überstände werden für 30 min bei 80 °C und 50 mbar eingeengt und getrocknet.

Für die Analyse von Glycerin, Diglycerinen, Triglycerinen und Tetraglycerinen mittels GC wird der Rückstand in 2 ml Pyridin:Chloroform (4:1) gelöst. 0,5 ml dieser Lösung werden mit 1 ml MSTFA [N-Methyl-N-(trimethylsilyl) trifluoroacetamide] versetzt. Die Alkohole werden durch Reaktion bei 80°C (30 Minuten) quantitativ in ihre Trimethylsilyether überführt und anschließend mittels GC/FID untersucht.
Dieses wird in einem Gaschromatograph, welcher mit einem split/splitless Injektor, einer Kapillarsäule und einem Flammenionisationdetektor ausgestattet ist, unter folgenden Bedingungen durchgeführt:

| | |
|---|---|
| Injektor: | 290 °C, Split 30 ml |

(fortgesetzt)

| Injektionsvolumen: | 1 μl |
| --- | --- |
| Säule: | 30 m *0,32 mm HP1 0,25 μm |
| Trägergas: | Helium, constant flow, 2 mL/min |
| Temperaturprogramm: | 80 °C - 300 °C mit 4 °C/min, dann 10 Minuten bei 300 °C konditionieren. |
| Detektor: | FID bei 310 °C |
| | Wasserstoff 35 ml/min |
| | Luft 240 ml/min |
| | Make Up Gas 12 ml/min |

Glycerin, Diglycerine, Triglycerine und Tetraglycerine werden aufgetrennt und ihr Massenanteil nach einer Methode des internen Standards bestimmt. Hierzu wird das GC System durch Vermessen von Mischungen der zu untersuchenden Glycerine und des internen Standards mit bekannter Zusammensetzung kalibriert.

Aus den Massenanteilen lässt sich das Massenverhältnis von Glycerin zu Diglycerin bestimmen sowie durch Subtraktion von 100% auch der Gehalt an Polyglycerinen mit einem Polymerisationsgrad von 2 und größer (100% minus Massenanteil des Glycerins), der Gehalt an Polyglycerinen mit einem Polymerisationsgrad von 3 und größer (100% minus Massenanteile des Glycerins und der Diglycerine), der Gehalt an Polyglycerinen mit einem Polymerisationsgrad von 4 und größer (100% minus Massenanteile des Glycerins, der Diglycerine und der Triglycerine) und der Gehalt an Polyglycerinen mit einem Polymerisationsgrad von 5 und größer (100% minus Massenanteile des Glycerins, der Diglycerine, der Triglycerine und der Tetraglycerine). Sollte in einem betrachteten Polyglycerin keine nachweisbare Menge an Diglycerin, jedoch Glycerin enthalten sein, so entspricht dies einem Massenverhältnis von Glycerin zu Diglycerin größer 1,4.

Eine erfindungsgemäß bevorzugte Zusammensetzung ist dadurch gekennzeichnet, dass der Polyglycerinester nach seiner vollständiger Hydrolyse im Mittel (Zahlenmittel) pro Mol Polyglycerinester

von 0,01 bis 0,07 Mol, bevorzugt von 0,01 bis 0,50 Mol, besonders bevorzugt von 0,01 bis 0,30 Mol, mindestens einer Carbonsäure a)

von 0,10 bis 1,70 Mol, bevorzugt von 0,30 bis 1,50 Mol, besonders bevorzugt von 0,40 bis 1,40 Mol, mindestens einer Carbonsäure b)

von 0,01 bis 0,80 Mol, bevorzugt von 0,01 bis 0,60 Mol, besonders bevorzugt von 0,05 bis 0,40 Mol, mindestens einer Carbonsäure c)

freisetzt.

Insbesondere ist sie dadurch gekennzeichnet, dass das Molverhältnis der nach vollständiger Hydrolyse des Polyglycerinesters erhaltenen Carbonsäure a) zu Carbonsäure b) zu Carbonsäure c)

0,6 bis 1,4 : 16,5 bis 20,5 : 3,0 bis 4,8, bevorzugt

0,8 bis 1,2 : 17,5 bis 19,5 : 3,5 bis 4,3, besonders bevorzugt

0,9 bis 1,1 : 18,0 bis 19,0 : 3,7 bis 4,1, beträgt.

Zur Bestimmung der Molverhältnisse lässt sich als Methode die oben beschriebene Methode einsetzen.

Es ist erfindungsgemäß bevorzugt, dass die Carbonsäuren a), b) und c) ausgewählt ist aus Fettsäuren, solche sind insbesondere ausgewählt aus linearen, gesättigten, unsubstituierten Carbonsäuren.

Insbesondere sind erfindungsgemäß Zusammensetzungen bevorzugt, die dadurch gekennzeichnet sind, dass die Carbonsäure a) ausgewählt ist aus Caprylsäure und Caprinsäure, die Carbonsäure b) ausgewählt ist aus Stearinsäure und Palmitinsäure und die Carbonsäure c) Behensäure ist.

Hydroxy-alkylmodifiziertes Guar ist vielfach beschrieben und beispielsweise als Esaflor HM 22 kommerziell erhältlich. Guar ist ein Galaktomannan, in welches langekttige Hydroxy-Alkylmodifikationen einfach durch Umsetzung mit Epoxyalkanen eingefügt werden können.

Erfindungsgemäß bevorzugte Zusammensetzungen enthalten hydroxy-alkylmodifziertes Guar, welches mit Alkylgruppen mit 16 bis 24, bevorzugt 18 bis 22, Kohlenstoffatomen modifiziert ist.

Es ist erfindungsgemäß bevorzugt, dass das hydroxy-alkylmodifzierte Guar zusätzlich hydroxypropyl modifiziert, ist. Ein erfindungsgemäß besonders bevorzugt enthaltenes Guar ist der nach INCI benannte Stoff C18-C22 Hydroxyalkyl Hydroxypropyl Guar. Hydroxy-alkylmodifiziertes Guar und solches, welches zusätzlich hydroxypropyl modifiziert ist, sowie Verfahren zur Herstellung dieser Verbindungen sind beispielsweise in der US4960876 beschrieben.

Die erfindungsgemäßen Zusammensetzungen sind hervorragend zur Formulierung von Deo oder AP-Deo-Zusammensetzungen (AP=Antiperspirant) geeignet, daher enthalten sie bevorzugt zusätzlich C) mindestens einen ausgewählt aus Deo-Wirkstoff und AP-Wirkstoff, insbesondere mindestens einen Deo-Wirkstoff und mindestens einen AP-Wirkstoff.

Es ist erfindungsgemäß bevorzugt, dass der AP-Wirkstoff ausgewählt ist aus der Gruppe umfassen, bevorzugt bestehend aus, Aluminium-Salzen und Zirkonium-Salzen. Es sind insbesondere erfindungsgemäße Zusammensetzungen bevorzugt, die dadurch gekennzeichnet sind, dass das Aluminium-Salz ausgewählt ist aus der Gruppe umfassend, bevorzugt bestehend aus:

Aluminum Acetate, Aluminum Behenate, Aluminum Benzoate, Aluminum Bromohydrate, Aluminum Butoxide, Aluminum Calcium Sodium Silicate, Aluminum Caprylate, Aluminum Capryloyl Hydrolyzed Collagen, Aluminum Chloride, Aluminiumchlorohydrat, Aluminum Chlorohydrex, Aluminum Chlorohydrex PEG, Aluminum Chlorohydrex PG, Aluminum Citrate, Aluminum Diacetate, Aluminum Dibenzoate/Stearate Hydroxide, Aluminum Dicetyl Phosphate, Aluminum Dichlorohydrate, Aluminum Dichlorohydrex PEG, Aluminum Dichlorohydrex PG, Aluminum Dilinoleate, Aluminum Dimyristate, Aluminum Distearate, Aluminum Glycinate, Aluminum Hydrogenated Tallow Glutamate, Aluminum Hydroxy Bis-Methylene Bis-Di-t-Butylphenyl Phosphate, Aluminum Iron Calcium Magnesium Germanium Silicates, Aluminum Iron Calcium Magnesium Zirconium Silicates, Aluminum Iron Silicates, Aluminum Isopropoxide, Aluminum Isostearate, Aluminum Isostearates/Laurates/Palmitates, Aluminum Isostearates/Laurates/Stearates, Aluminum Isostearates/Myristates, Aluminum Isostearates/Palmitates, Aluminum Isostearates/Stearates, Aluminum Isostearyl Glyceryl Phosphate, Aluminum Laccate, Aluminum Lactate, Aluminum Lanolate, Aluminum/Magnesium Hydroxide Stearate, Aluminum Magnesium Oxide, Aluminum Methionate, Aluminum Myristate, Aluminum Myristates/Palmitates, Aluminum PCA, Aluminum Phenolsulfonate, Aluminum Sesquichlorohydrate, Aluminum Sesquichlorohydrex PEG, Aluminum Sesquichlorohydrex PG, Aluminum Starch Octenylsuccinate, Aluminum Stearate, Aluminum Stearates, Aluminum Stearoyl Glutamate, Aluminum Sucrose Octasulfate, Aluminum Sulfate, Aluminum Triformate, Aluminum Triphosphate, Aluminum Tristearate, Aluminum Undecylenoyl Collagen Amino Acids, Aluminum Zinc Oxide, Aluminum Zirconium Trichlorohydrate, Aluminum Zirconium Trichlorohydrex GLY, Aluminum Zirconium Tetrachlorohydrate, Aluminium Zirconium Tetrachlorohydrex GLY, Aluminum Zirconium Tetrachlorohydrex PEG, Aluminum Zirconium Tetrachlorohydrex PG, Aluminium Zirconium Pentachlorohydrat, Aluminum Zirconium Pentachlorohydrex GLY, Aluminum Zirconium Octachlorohydrate, Aluminum Zirconium Octachlorohydrex GLY, Ammonium Alum, Ammonium Silver Zinc Aluminum Silicate, Calcium Aluminum Borosilicate, Cobalt Aluminum Oxide, Magnesium/Aluminum/Hydroxide/Carbonate, Magnesium Aluminum Silicate, Magnesium/Aluminum/Zinc/Hydroxide/Carbonate, Potassium Alum, Potassium Aluminum Polyacrylate, Silver Magnesium Aluminum Phosphate, Sodium Alum, Sodium Aluminate, Sodium Aluminum Chlorohydroxy Lactate, Sodium/Aluminum Hydroxide/Oxalate/Sulfate, Sodium/Aluminum/Iron Hydroxide/Oxalate/Sulfate, Sodium/Aluminum/Iron/Sulfate/Citrate/Hydroxide, Sodium/Aluminum/Iron/Sulfate/Oxalate/Hydroxide, Sodium/Aluminum/Iron/Sulfate/Tartarate/Hydroxide, Sodium Aluminum Lactate, Sodium Phosphorus/Zinc/Calcium/Silicon/Aluminum/Silver Oxides, Sodium Potassium Aluminum Silicate, Sodium Silicoaluminate, Sodium Silver Aluminum Silicate, Tromethamine Magnesium Aluminum Silicate und Alaun, insbesondere Aluminiumchlorohydrat.

Unter dem Begriff "Aluminiumchlorohydrat" werden im Zusammenhang mit der vorliegenden Erfindung die Salze der allgemeinen Summenformel $Al_nCl_{(3n-m)}(OH)_m$, insbesondere $Al_2Cl(OH)_5$, verstanden.

Geeignete Zirkonium-Salze sind ausgewählt aus den in US20070071701, US3792068 und US2854382 beschriebenen zirkoniumhaltigen Verbindungen.

Geeignete Deo-Wirkstoffe sind ausgewählt aus der Gruppe umfassend, bevorzugt bestehend aus den in EP2421614 genannten Deodorantien und keimhemmenden Mitteln sowie Zink-Salzen.

Erfindungsgemäß bevorzugt ist das Zink-Salz ausgewählt aus der Gruppe der Zink-Salze der Essigsäure, Milchsäure, Oxalsäure, Bernsteinsäure, Fumarsäure, Maleinsäure, Ricinolsäure und/oder Citronensäure gewählt werden.

Die erfindungsgemäßen Zusammensetzungen stabilisieren insbesondere Emulsionen, so dass eine erfindungsgemäß bevorzugte Zusammensetzung dadurch gekennzeichnet ist, dass sie eine Emulsion, insbesondere eine Öl-in-Wasser-Emulsion ist.

Erfindungsgemäß bevorzugte Emulsion enthalten bezogen auf die Gesamtemulsion von 0,1 Gew.-% bis 60,0 Gew.-%, bevorzugt von 4,0 Gew.-% bis 30,0 Gew.-%, besonders bevorzugt von 5,0 Gew.-% bis 20,0 Gew.-%, mindestens ein Öl, insbesondere ein kosmetisches Öl.

Geeignete kosmetische Öle sind beispielsweise in DE10361202 aufgeführt.

Zur guten Emulsionsstabilität ist es bevorzugt, dass die erfindungsgemäßen Emulsionen bezogen auf die Gesamtemulsion von 0,1 Gew.-% bis 15,0 Gew.-%, bevorzugt von 0,5 Gew.-% bis 10,0 Gew.-%, besonders bevorzugt von 1,0 Gew.-% bis 7,0 Gew.-%, mindestens eines Emulgators enthalten.

Bevorzugt enthaltene Emulgatoren sind insbesondere Öl-in-Wasser-Emulgatoren, insbesondere in EP2421614 aufgeführte Emulgatoren.

Die erfindungsgemäßen Zusammensetzungen stabilisieren auch sonst schwierige Formulierungen, die eine niedrige Viskosität aufweisen, somit ist eine erfindungsgemäß bevorzugte Zusammensetzung dadurch gekennzeichnet, dass sie eine Viskosität in einem Bereich von 500 bis 20000, bevorzugt von 1500 bis 10000 mPas, aufweist, wobei die Viskosität bei 25 °C mit Brookfield RVT, Spindel 4, 5 UpM, gemessen wird.

Insbesondere sind Zusammensetzungen bevorzugt, die im Wesentlichen polyglycoletherfrei und im Wesentlichen frei von alkoxylierten Verbindungen sind. Unter dem Begriff "im Wesentlichen frei von alkoxylierten Verbindungen" und "im Wesentlichen polyglycoletherfrei" im Zusammenhang mit der vorliegenden Erfindung ist zu verstehen, dass die Zusammensetzungen, mit gegebenenfalls der erfindungsgemäß enthaltenen Komponente B) als Ausnahme, keine nennenswerten Mengen an alkoxylierten oder polyglcolether enthaltende Verbindungen aufweist, die eine oberflächenaktive Wirkung ausüben. Insbesondere ist hierunter zu verstehen, dass diese Verbindungen in Mengen von kleiner 1 Gew.-%, bevorzugt von kleiner 0,1 Gew.-%, besonders bevorzugt von kleiner 0,01 Gew.-% bezogen auf die Gesamtzusammensetzung, insbesondere keine nachweisbaren Mengen, enthalten sind.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

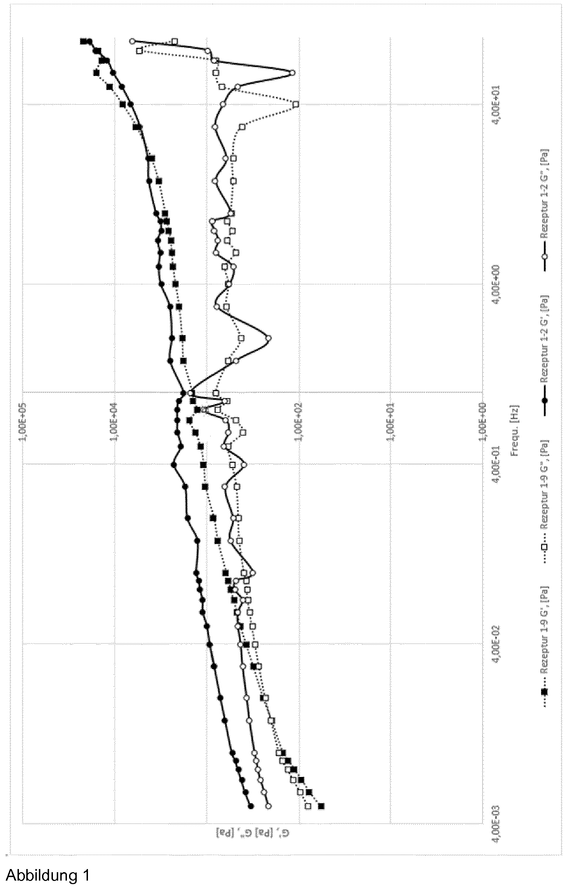

Abbildung 1 zeigt Speichermodul und Verlustmodul für die nach erfindungsgemäßer Rezeptur 1-2 bzw. nicht erfindungsgemäßer Rezeptur 1-9 hergestellten Emulsionen.

Beispiele:

*Vorsynthesebeispiel 1 (Glycerylmonobehenat)*

Ein Gemisch aus Glycerin (92 g, 1.0 mol), Behensäure (234.6 g, 0.69 mol) und $Ca(OH)_2$ (0.06 g) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.5 erreicht war. Nach Abkühlen auf 90 °C wurde anschließend eine 5%ige Lösung von $H_3PO_4$ in Glycerin (2 g) zugegeben und das Reaktionsgemisch wieder auf 240 °C erhitzt. Ab einer Temperatur von ≥100 °C wurde der Druck bei gleichzeitiger Stickstoffeinleitung auf 10 mbar gesenkt und so lange destilliert, bis kein Destillat mehr anfiel. Nach Zugabe eines Filterhilfsmittels wird über einen Filter filtriert.

*Vorsynthesebeispiel 2 (Glycerylmonostearat)*

Ein Gemisch aus Glycerin (198,3 g, 2,15 mol), Stearinsäure und Palmitinsäure im Verhältnis 1:1 (401.8 g, 1.49 mol) und $Ca(OH)_2$ (0.13 g) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.5 erreicht war. Nach Abkühlen auf 90 °C wurde anschließend eine 5%ige Lösung von $H_3PO_4$ in Glycerin (4.3 g) zugegeben und das Reaktionsgemisch wieder auf 240 °C erhitzt. Ab einer Temperatur von ≥100 °C wurde der Druck bei gleichzeitiger Stickstoffeinleitung auf 10 mbar gesenkt und so lange destilliert, bis kein Destillat mehr anfiel. Nach Zugabe eines Filterhilfsmittels wird über einen Filter filtriert.

*Vorsynthesebeispiel 3 (Polyglyceryl-4-Stearat)*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4830 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 990 mg KOH/g aufwies.

Ein Gemisch aus dem so erhaltenen Polyglycerin (252,2 g, 0,8 mol) und Stearinsäure und Palmitinsäure im Verhältnis 1:1 (97,8 g, 0,36 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war und das Gemisch bei 240 °C klar und homogen war.

*Vorsynthesebeispiel 4 (Polyglyceryl-3-Caprylat/Caprat)*

Ein Gemisch aus kommerziell erhältlichem Polyglycerin-3 (Solvay; 240 g, 1 mol) und Caprylsäure und Caprinsäure im Verhältnis 60:40 (78,8 g, 0,5 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤0.5 erreicht war.

*Synthesebeispiel 1 (nicht erfindungsgemäß)*

Ein Gemisch aus Estern erhalten wie beschrieben in Vorsynthesebeispiel 1 (22,5 g), in Vorsynthesebeispiel 2 (34,5 g), in Vorsynthesebeispiel 3 (88,5 g) und in Vorsynthesebeispiel 4 (4,5 g) wurde auf 80 °C erhitzt und das Gemisch anschließend für 3 h bei dieser Temperatur gerührt..

Der so erhaltene Polyglycerinester weist nach seiner vollständigen Hydrolyse einen Polymerisationsgrad des Polyglycerins von <3 auf.

*Vorsynthesebeispiel 5 (Polyglyceryl-6-Stearat)*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4830 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 960 mg KOH/g aufwies.
Ein Gemisch aus dem so erhaltenen Polyglycerin (1008,7 g, 2,2 mol) und Stearinsäure und Palmitinsäure im Verhältnis 1:1 (391,3 g, 1,5 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war und das Gemisch bei 240 °C klar und homogen war.

*Synthesebeispiel 2 (erfindungsgemäß)*

Ein Gemisch aus Estern erhalten wie beschrieben in Vorsynthesebeispiel 1 (18,75 g), in Vorsynthesebeispiel 2 (32,25 g), in Vorsynthesebeispiel 5(94,5 g) und in Vorsynthesebeispiel 4 (4,5 g) wurde auf 80 °C erhitzt und das Gemisch anschließend für 3 h bei dieser Temperatur gerührt..
Der so erhaltene Polyglycerinester weist nach seiner vollständigen Hydrolyse einen Polymerisationsgrad des Polyglycerins von ca. 3,9 auf.

*Vorsynthesebeispiel 6 (Polyglyceryl-10-Stearat)*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4830 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 875 mg KOH/g aufwies.
Ein Gemisch aus dem so erhaltenen Polyglycerin (252,2 g, 0,33 mol) und Stearinsäure und Palmitinsäure im Verhältnis 1:1 (97,8 g, 0,36 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war und das Gemisch bei 240 °C klar und homogen war.

*Vorsynthesebeispiel 7 (Polyglyceryl-10-Caprylat/Caprat)*

Ein Polyglycerin erhalten wie beschrieben in Beispiel 5a (240 g; 0,32 mol) und Caprylsäure und Caprinsäure im Verhältnis 60:40 (78,8 g, 0,5 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war.

*Synthesebeispiel 3 (erfindungsgemäß)*

Ein Gemisch aus Estern erhalten wie beschrieben in Vorsynthesebeispiel 1 (18,8 g), in Vorsynthesebeispiel 2 (32,7 g), in Vorsynthesebeispiel 6 (93,8 g) und in Vorsynthesebeispiel 7(4,7 g) wurde auf 80 °C erhitzt und das Gemisch anschließend für 3 h bei dieser Temperatur gerührt.
Der so erhaltene Polyglycerinester weist nach seiner vollständigen Hydrolyse einen Polymerisationsgrad des Polyglycerins von ca. 4,5 auf.

*Vorsynthesebeispiel 8*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4830 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 924 mg KOH/g aufwies.
Ein Gemisch aus dem so erhaltenen Polyglycerin (231,9 g, 0,296 mol) und Stearinsäure und Palmitinsäure im Verhältnis 1:1 (85,42 g, 0,32 mol) und Caprylsäure und Caprinsäure im Verhältnis 60:40 (3,82 g, 0,025 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war und das Gemisch bei 240 °C klar und homogen war.

*Synthesebeispiel 4 (erfindungsgemäß)*

Ein Gemisch aus Estern erhalten wie beschrieben in Vorsynthesebeispiel 1 (18,75 g), in Vorsynthesebeispiel 2 (33,75 g) und in Vorsynthesebeispiel 8(97,5 g) wurde auf 80 °C erhitzt und das Gemisch anschließend für 3 h bei dieser Temperatur gerührt. Der so erhaltene Polyglycerinester weist nach seiner vollständigen Hydrolyse einen Polymerisationsgrad des Polyglycerins von ca. 3,6 auf.

*Vorsynthesebeispiel 9*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4830 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 884 mg KOH/g aufwies.
Ein Gemisch aus dem so erhaltenen Polyglycerin (243,5 g, 0,32 mol) und Stearinsäure und Palmitinsäure im Verhältnis 1:1 (85,42 g, 0,32 mol) und Caprylsäure und Caprinsäure im Verhältnis 60:40 (3,82 g, 0,025 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war und das Gemisch bei 240 °C klar und homogen war.

*Synthesebeispiel 5 (erfindungsgemäß)*

Ein Gemisch aus Estern erhalten wie beschrieben in Vorsynthesebeispiel 1 (18,75 g), in Vorsynthesebeispiel 2 (33,75 g) und in Vorsynthesebeispiel 8(97,5 g) wurde auf 80 °C erhitzt und das Gemisch anschließend für 3 h bei dieser Temperatur gerührt. Der so erhaltene Polyglycerinester weist nach seiner vollständigen Hydrolyse einen Polymerisationsgrad des Polyglycerins von ca. 3,8 auf.

*Anwendungsbeispiele erfindungsgemäßer Zusammensetzungen vs. nicht erfindungsgemäßer Zusammensetzungen:*

Alle Konzentrationen in den Anwendungsbeispielen sind in Gewichtsprozent angegeben. Zur Herstellung der Emulsionen wurden dem Fachmann bekannte übliche Homogenisierverfahren eingesetzt.
Die Herstellung der Emulsionen erfolgte daher typischerweise so, dass Öl- und Wasserphase auf 70 - 75°C erwärmt wurden. Anschließend wurde entweder die Ölphase in die Wasserphase eingerührt oder Öl- und Wasserphase ohne Rühren zusammengegeben. Anschließend wurde mit einem geeigneten Homogenisator (z.B. Ultra Turrax) für ca. 1-2 Minuten homogenisiert.
Stabilisierende Polymere wurden entweder als Bestandteil der Ölphase (z.B. Guarderivate) oder als wässrige Suspension (z.B. Cellulosederivate) bei Temperaturen von 50 - 60°C in die Emulsion eingerührt. Anschließend wurde kurz homogenisiert. Zugabe weiterer Inhaltsstoffe (z.B. Konservierungsmittel, Wirkstoffe) erfolgte bevorzugt bei 40°C. Falls die Rezepturen mit organischen Säuren konserviert wurden, wurde der pH-Wert der Emulsionen auf ca. 5 eingestellt.

1) Differenzierung der Performance gegen den Stand der Technik

Diese Versuche zeigen, dass die erfindungsgemäßen Emulgatoren Vorteile in Bezug auf Emulsionsstabilität aufweisen. Als Repräsentanten für PEG-freie O/W Emulgator wurden dabei die Kombination aus Methyglucose Sesquistearat / Polyglyceryl-4 Laurat sowie Polyglyceryl-4 Laurate/Succinate (and) Aqua gewählt.
Zur Überprüfung der Lagerstabilität der Emulsionen wurden diese drei Monate bei Raumtemperatur und 40°C gelagert. Zur Überprüfung der Kältestabilität wurde außerdem ein Monat lang bei -5°C gelagert und drei Gefrier-Tau-Zyklen von 25°C/-15°C/25°C durchgeführt. Deutliche Veränderungen im Erscheinungsbild oder der Konsistenz sowie insbesondere Öl- oder Wasserabscheidungen wurden als Kriterien für Instabilität gewichtet.

A) Aluminiumsalzhaltige AP/Deo Formulierung

| Rezeptur | 1-1 | 1-2 | 1-3 | 1-4 | 1-5 | 1-6 | 1-7 | 1-8 | 1-9 |
|---|---|---|---|---|---|---|---|---|---|
| | Nicht erfind-ungsgem. | erfindungs-gem. | erfindungs-gem. | Nicht erfind-ungsgem. | Nicht erfind-ungsgem. | Nicht erfind-ungsgem. | Nicht erfind-ungsgem. | Nicht erfind-ungsgem. | Nicht erfind-ungsgem. |
| Nicht erfindungs-gem. Emulgator nach Synthese-beispiel 1 | 3,2 | | | | | | | | |
| Erfindungsgem. Emulgator nach Synthesebeispiel 2 | | 3,2 | | | | | | | 3,2 |
| Erfindungsgem. Emulgator nach Synthesebeispiel 3 | | | 3,2 | | | | | | |
| Methylglucose Sesquistearate[1] | | | | 1,75 | 1,75 | | | | |
| Polyglyceryl-4 Laurate[2] | | | | 0,25 | 0,25 | | | | |
| Polyglyceryl-4 Lau-rate/Succinate (and) Aqua[3] | | | | | | 2,1 | 2,1 | | |
| Polyglyceryl-6 Stea-rate; Polyglyceryl-6 Behenate [29] | | | | | | | | 3,2 | |
| C18-C22 Hydroxy-alkyl Hydroxypropyl Guar[4] | 0,2 | 0,2 | 0,2 | 0,5 | | 0,5 | | 0,2 | |
| Hydroxyethylcellu-lose [5] | | | | | 0,5 | | 0,5 | | |
| Hydroxypropyl Guar[30] | | | | | | | | | 0,2 |
| Isoamyl Cocoate[6] | 5,4 | 5,4 | 5,4 | 7,0 | 7,0 | 5,4 | 5,4 | 5,4 | 5,4 |
| Wasser | Ad 100 | Ad 100 | Ad 100 | Ad 100 | Ad 100 | Ad 100 | Ad 100 | Ad 100 | Ad 100 |

EP 3 192 491 B1

(fortgesetzt)

| Rezeptur | 1-1 | 1-2 | 1-3 | 1-4 | 1-5 | 1-6 | 1-7 | 1-8 | 1-9 |
|---|---|---|---|---|---|---|---|---|---|
| | Nicht erfind-ungsgem. | erfindungs-gem. | erfindungs-gem. | Nicht erfind-ungsgem. | Nicht erfind-ungsgem. | Nicht erfind-ungsgem. | Nicht erfind-ungsgem. | Nicht erfind-ungsgem. | Nicht erfind-ungsgem. |
| Aluminum Chloro-hydrate (50% aq.)[7] | 20,0 | 20,0 | 20,0 | 15,0 | 15,0 | 20,0 | 20,0 | 20,0 | 20,0 |
| Methylisothia-zolinone, Methylpa-raben, Ethylpa-raben; Dipropylene Glycol[8] | 0,8 | 0,8 | 0,8 | 0,8 | 0,8 | 0,8 | 0,8 | 0,8 | 0,8 |
| Ist-pH | 4,2 | 4,2 | 4,2 | 4,2 | 4,2 | 4,2 | 4,2 | 4,2 | 4,2 |
| Viskosität [Pas] (Brookfield RVT, Spindel 4, 5 UpM) | 21 | 5 | 9 | 22 | 16 | "Wasserdünn" | "Wasserdünn" | 7 | "Wasserdünn" |
| Stabilität | Nicht stabil, Wasserseparation nach 2 Tagen bei RT | stabil | stabil | Nicht stabil, Wassersepara-tion nach 2 Wo-chen bei RT | Nicht stabil, Wasserseparation nach 2 Tagen bei RT | Nicht stabil, Wasserseparation nach 2 Tagen bei RT | Nicht stabil, Wasserseparation nach 2 Tagen bei RT | Nicht stabil, Wasserseparation nach 1 Monat bei 40°C, bzw. nach 1 Monat bei - 5°C | Nicht stabil, Wasserseparation nach 2 Tagen bei RT |

[1] TEGO Care PS (Evonik Industries AG)
[2] TEGO Care PL 4 (Evonik Industries AG)
[29] Polyglycerinester entsprechend Synthesebeispiel 1 in EP2705832
[30] ESAFLOR HDR (Lamberti S.p.A.)
[3] NatraGem E145 (Croda Int. Plc)
[4] ESAFLOR HM 22 (Lamberti S.p.A.)
[5] Natrosol 250 HHR (Ashland Specialty Ingredients)
[6] TEGOSOFT AC (Evonik Industries AG)
[7] Locron LIC (Clariant AG)
[8] Microcare MEM (Thor)

B) Aluminiumfreie Deo-Formulierung ohne AP-Wirkstoffe

| Rezeptur | 2-1 | 2-2 | 2-3 | 2-4 | 2-5 | 2-6 | 2-7 | 2-8 | 2-9 |
|---|---|---|---|---|---|---|---|---|---|
| | Nicht erf. Gem. | Erf. Gem. | Erf. Gem. | Nicht erf. Gem. | Nicht erf. Gem. | Nicht erf. Gem. | Nicht erf. Gem. | Nicht erf. Gem. | Nicht erf. Gem. |
| Nicht erfindungsgem. Emulgator nach Synthesebeispiel 1 | 3,2 | | | | | | | | |
| Erfindungsgem. Emulgator nach Synthesebeispiel 2 | | 3,2 | | | | | | | |
| Erfindungsgem. Emulgator nach Synthesebeispiel 3 | | | 3,2 | | | | | | 3,2 |
| Methylglucose Sesquistearate[1] | | | | 2,8 | 2,8 | | | | |
| Polyglyceryl-4 Laurate[2] | | | | 0,4 | 0,4 | | | | |
| Polyglyceryl-4 Laurate/Succinate (and) Aqua [3] | | | | | | 3,2 | 3,2 | | |
| Polyglyceryl-6 Stearate; Polyglyceryl-6 Behenate [29] | | | | | | | | 3,2 | |
| C18-C22 Hydroxyalkyl Hydroxypropyl Guar[4] | 0,15 | 0,15 | 0,15 | 0,15 | | 0,15 | | 0,15 | |
| Hydroxyethylcellulose [5] | | | | | 0,15 | | 0,15 | | |
| Hydroxypropyl Guar[30] | | | | | | | | | 0,15 |
| Polyglyceryl-3 Caprylate[9] | 0,5 | 0,5 | 0,5 | 0,5 | 0,5 | 0,5 | 0,5 | 0,5 | 0,5 |
| Zinc Ricinoleate[10] | 1,0 | 1,0 | 1,0 | 1,0 | 1,0 | 1,0 | 1,0 | 1,0 | 1,0 |

(fortgesetzt)

| Rezeptur | 2-1 | 2-2 | 2-3 | 2-4 | 2-5 | 2-6 | 2-7 | 2-8 | 2-9 |
|---|---|---|---|---|---|---|---|---|---|
| | Nicht erf. Gem. | Erf. Gem. | Erf. Gem. | Nicht erf. Gem. | Nicht erf. Gem. | Nicht erf. Gem. | Nicht erf. Gem. | Nicht erf. Gem. | Nicht erf. Gem. |
| Caprylic/Capric Triglyceride | 5,65 | 5,65 | 5,65 | 5,65 | 5,65 | 5,65 | 5,65 | 5,65 | 5,65 |
| Wasser | Ad 100 | Ad 100 | Ad 100 | Ad 100 | Ad 100 | Ad 100 | Ad 100 | Ad 100 | Ad 100 |
| Glycerin | 3,0 | 3,0 | 3,0 | 3,0 | 3,0 | 3,0 | 3,0 | 3,0 | 3,0 |
| Benzyl Alcohol, Benzoic Acid, Sorbic Acid[11] | 1,0 | 1,0 | 1,0 | 1,0 | 1,0 | 1,0 | 1,0 | 1,0 | 1,0 |
| Zitronensäure (50% aq.) | q.s. | q.s. | q.s. | q.s. | q.s. | q.s. | q.s. | q.s. | q.s. |
| pH | 5,0 | 5,0 | 5,0 | 5,0 | 5,0 | 5,0 | 5,0 | 5,0 | 5,0 |
| Viskosität [Pas] (Brookfield RVT, Spindel 4,5 UpM) | 7 | 4 | 5 | 18 | 10 | "was serdü nn" | "wass erdün n" | 2 | 2 |
| Stabilität | Nicht stabil, Wasserseparation nach 3 Monaten bei 40°C | stabil | stabil | zu hohe Viskosität, nicht gerfrierstabil (-5°C und -15°C) | Nicht stabil, Wasserseparation bei Wärmelagerung (40°C) | Nicht stabil, Phasentrennung nach einem Tag | Nicht stabil, Phasentrennung nach einem Tag | Nicht stabil, Wasserseparation nach 3 Monaten bei RT | Nicht stabil Wasserseparation nach 1 Monat bei 40°C |

[9] TEGO Cosmo P 813 (Evonik Industries AG)
[10] TEGODEO PY 88 G (Evonik Industries AG)
[11] Rokonsal BSB-N (Ashland Specialty Ingredients)

C) O/W Deodorant-Emulsion enthaltend Kalialaun

| Rezeptur | 3-1 | 3-2 | 3-3 | 3-4 | 3-5 | 3-6 | 3-7 | 3-8 | 3-9 |
|---|---|---|---|---|---|---|---|---|---|
| | Nicht Erf. Gem. | Erf. Gem. | Erf. Gem. | Nicht Erf. Gem. | Nicht Erf. Gem. | Nicht Erf. Gem. | Nicht Erf. Gem. | Nicht Erf. Gem. | Nicht Erf. Gem. |
| Nicht erfindungsgem. Emulgator nach Synthesebeispiel 1 | 4,8 | | | | | | | | |
| Erfindungsgem. Emulgator nach Synthesebeispiel 2 | | 4,8 | | | | | | | 4,8 |
| Erfindungsgem. Emulgator nach Synthesebeispiel 3 | | | 4,8 | | | | | | |
| Methylglucose Sesquistearate[1] | | | | 4,2 | 4,2 | | | | |
| Polyglyceryl-4 Laurate[2] | | | | 0,6 | 0,6 | | | | |
| Polyglyceryl-4 Laurate/Succinate (and) Aqua [3] | | | | | | 4,8 | 4,8 | | |
| Polyglyceryl-6 Stearate; Polyglyceryl-6 Behenate [29] | | | | | | | | 4,8 | |
| C18-C22 Hydroxyalkyl Hydroxypropyl Guar[4] | 0,25 | 0,25 | 0,25 | 0,25 | | 0,25 | | 0,25 | |
| Hydroxyethylcellulose [5] | | | | | 0,25 | | 0,25 | | |
| Hydroxypropyl Guar[30] | | | | | | | | | 0,25 |
| Isopropyl Palmitate | 5,0 | 5,0 | 5,0 | 5,0 | 5,0 | 5,0 | 5,0 | 5,0 | 5,0 |
| Wasser | Ad 100 | Ad 100 | Ad 100 | Ad 100 | Ad 100 | Ad 100 | Ad 100 | Ad 100 | Ad 100 |
| Glycerin | 3,0 | 3,0 | 3,0 | 3,0 | 3,0 | 3,0 | 3,0 | 3,0 | 3,0 |

(fortgesetzt)

| Rezeptur | 3-1 | 3-2 | 3-3 | 3-4 | 3-5 | 3-6 | 3-7 | 3-8 | 3-9 |
|---|---|---|---|---|---|---|---|---|---|
| | Nicht Erf. Gem. | Erf. Gem. | Erf. Gem. | Nicht Erf. Gem. | Nicht Erf. Gem. | Nicht Erf. Gem. | Nicht Erf. Gem. | Nicht Erf. Gem. | Nicht Erf. Gem. |
| Kalialaun | 5,0 | 5,0 | 5,0 | 5,0 | 5,0 | 5,0 | 5,0 | 5,0 | 5,0 |
| Methylisothiazolinone, Methylparaben, Ethylparaben; Dipropylene Glycol[8] | 0,8 | 0,8 | 0,8 | 0,8 | 0,8 | 0,8 | 0,8 | 0,8 | 0,8 |
| Ist-pH | 3,4 | 3,4 | 3,4 | 3,4 | 3,4 | 3,4 | 3,4 | 3,4 | 3,4 |
| Viskosität [Pas] (Brookfield RVT, Spindel 4, 5 UpM) | 11 → 23 | 3 | 3 | 4 | - | - | - | 11 → 34 | - |
| Stabilität | zu starker Viskositäts anstieg währ end Lagerung | stabil | stabil | Nicht stabil, Wasserseparation nach 1 Monatbei 40°C | Nicht stabil, Phas entre nnung 3h nach Herstellung | Nicht stabil, Phas entre nnung 3h nach Herstellung | Nicht stabil, Phas entre nnung 3h nach Herstellung | zu starker Viskositätsanstieg währ end Lagerung | Nicht stabil, Phas entre nnung 3h nach Herstellung |

In allen drei Vergleichsformulierungen aus dem Anwendungsbereich AP/Deo- bzw. Deo-Roll-on, welche verschiedene Wirkstoffe enthalten, erlauben die erfindungsgemäßen Emulgatoren die Formulierung einer stabilen Emulsion, wohingegen der nicht erfindungsgemäße Emulgator und die Repräsentanten des Stand der Technik keine stabile Emulsion ermöglichen. Die Verwendung von nicht erfindungsgemäß modifiziertem Guar führt ebenfalls zu nicht lagerstabilen Emulsionen.

Formulierungen 1-2 und 1-9 wurden hinsichtlich ihrer Ausbildungsfähigkeit einer Fließgrenze untersucht. Die Messungen wurden mit einem Rheometer von Anton Paar, Modell MCR 301, Platte - Platte (40 mm) Geometrie mit einem Spalt von 1 mm bei einer Temperatur von 25°C und 1 bar Druck durchgeführt. Bestimmt wurden Speichermodul und Verlustmodul für die nach erfindungsgemäßer Rezeptur 1-2 bzw. nicht erfindungsgemäßer Rezeptur 1-9 hergestellten Emulsionen. Die Proben wurden bei konstanter Belastung von 0,20 Pa über den Frequenzbereich von 0,005 bis 90 Hz vermessen. Die rheologische Fließgrenze ist dadurch definiert, dass im vermessenen Frequenzbereich das Speichermodul (G') stets höhere Werte aufweist als das Verlustmodul (G") (siehe Abb. 1).

Weitere Anwendungsbeispiele außerhalb des AP/Deo Bereichs.

Diese Beispiele zeigen, dass die erfindungsgemäßen Zusammensetzungen in einer Vielzahl kosmetischer Formulierungen eingesetzt werden können.

*Sun Care Spray SPF 30*

| Rezeptur | 4 |
|---|---|
| erfindungsgemäßer Emulgator nach Synthesebeispiel 4 | 4,0 |
| Phenoxyethyl Caprylate [12] | 3,2 |
| Isopropyl Palmitate | 2,0 |
| Bis-Ethylhexyloxyphenol Methoxyphenyl Triazine [13] | 3,0 |
| Butyl Methoxydibenzoylmethane | 2,0 |
| EHC | 2,0 |
| Ethylhexylsalicylat | 4,0 |
| Octocrylene | 4,0 |
| Glycerin | 3,0 |
| Wasser | ad 100 |
| Carbomersuspension 1[14] | 1,0 |
| Tris(hydroxymethyl)-aminomethan (30% aq.) | 0,6 |
| UV-Filterlösung [15] | 10,0 |
| Methylisothiazolinone, Methylparaben, Ethylparaben; Dipropylene Glycol[8] | 0,8 |

[11] TEGOSOFT XC (Evonik Industries AG)
[13] Tinosorb S (BASF SE)
[14] Acrylates/C10-30 Alkyl Acrylate Crosspolymer (TEGO Carbomer 341ER, Evonik Industries AG) 20%ig in Phenoxyethyl Caprylate
[15] 20 % Phenylbenzimidazole Sulfonic Acid, 8,8% Tris (hydroxymethyl)-aminomethan, demineralisiertes Wasser ad 100%

*Körperlotion*

| Rezeptur | 5 |
|---|---|
| erfindungsgemäßer Emulgator nach Synthesebeispiel 5 | 4,0 |
| Isoamyl Cocoate [6] | 2,5 |
| Caprylic/Capric Triglyceride | 3,5 |
| Wasser | ad 100 |
| Creatine [16] | 0,5 |

(fortgesetzt)

| | |
|---|---|
| Carbomersuspension 2 [17] | 1,0 |
| Natriumhydroxid (10% aq.) | 0,6 |
| Phenoxyethanol, Ethylhexylglycerin [18] | 0,7 |

[16] TEGO Cosmo C 100 (Evonik Industries AG)
[17] Carbomer (TEGO Carbomer 141, Evonik Industries AG) 20%ig in Ethylhexyl Stearate
[18] Euxyl PE 9010 (Schülke & Mayr GmbH)

*Natürliche Pflegecreme*

| Rezeptur | 6 |
|---|---|
| erfindungsgemäßer Emulgator nach Synthesebeispiel 2 | 6,0 |
| Caprylic/Capric Triglyceride | 8,0 |
| Isopropyl Palmitate | 11,0 |
| Prunus Amygdalus Dulcis (Sweet Almond) Oil | 10,0 |
| Wasser | ad 100 |
| Glycerin | 3,0 |
| Natriumhydroxid (10% aq.) | 0,2 |
| Benzyl Alcohol, Benzoic Acid, Sorbic Acid [11] | 0,8 |

*Anti-Aging Creme*

| Rezeptur | 7 |
|---|---|
| erfindungsgemäßer Emulgator nach Synthesebeispiel 4 | 6,0 |
| Caprylic/Capric Triglyceride | 9,5 |
| C12-15 Alkyl Benzoate | 9,5 |
| Wasser | ad 100 |
| Glycerin | 3,0 |
| Tetrapeptide-21; Glycerin; Butylene Glycol; Aqua [19] | 4,0 |
| Sodium Hyaluronate [20] | 0,1 |
| Hydrolyzed Hyaluronic Acid [21] | 0,1 |
| Aqua; Ethylhexyl Stearate; Sodium Hyaluronate Cross polymer; Polyglyceryl-4 Diisostearate/ Polyhydroxysteara te/Sebacate; Sodium Isostearate [22] | 5,0 |
| Methylisothiazolinone, Methylparaben, Ethylparaben; Dipropylene Glycol[8] | 0,8 |

[19] TEGO PEP 4-17 (Evonik Industries)
[20] HyaCare (Evonik Industries)
[21] HyaCare 50 (Evonik Industries)
[22] HyaCare Filler CL (Evonik Industries)

*O/W Foundation*

| Rezeptur | 8 |
|---|---|
| erfindungsgemäßer Emulgator nach Synthesebeispiel 3 | 6,0 |

(fortgesetzt)

| | |
|---|---|
| Myristyl Myristate | 2,0 |
| Isopropyl Myristate | 6,0 |
| Decyl Cocoate | 6,0 |
| Cetyl Ricinoleate | 1,0 |
| Wasser | ad 100 |
| Glycerin | 1,0 |
| Titanium Dioxide [23] | 8,0 |
| Iron Oxides [24] | 0,9 |
| Iron Oxides [25] | 0,2 |
| Iron Oxides [26] | 0,4 |
| Iron Oxides [27] | 0,1 |
| Cellulose [28] | 2,0 |
| Natriumhydroxid (10% aq.) | 0,2 |
| Benzyl Alcohol, Benzoic Acid, Sorbic Acid [11] | 1,0 |
| [23] Hombitan AC 360 (Sachtleben) [24] Sicovit Gelb 10 E 172 (Rockwood Pigments) [25] Sicovit Rot 30 E 172 (Rockwood Pigments) [26] Sicovit Braun 70 E 172 (Rockwood Pigments) [27] Sicovit Schwarz 80 E 172 (Rockwood Pigments) [28] TEGO Feel Green (Evonik Industries AG) | |

**Patentansprüche**

1. Zusammensetzung enthaltend

A) Polyglycerinester,
der nach seiner vollständiger Hydrolyse

a) mindestens eine Carbonsäure mit 6 bis 14, bevorzugt 8 bis 12, Kohlenstoffatomen
b) mindestens eine Carbonsäure mit 16 bis 20, bevorzugt 18 bis 20, Kohlenstoffatomen
c) mindestens eine Carbonsäure mit 22 bis 28, bevorzugt 22 bis 24, Kohlenstoffatomen

freisetzt und
der nach seiner vollständiger Hydrolyse ein Polyglycerin freisetzt, das einen mittleren Polymerisationsgrad von 3,0 bis 5,0, bevorzugt von 3,3 bis 4,7, besonders bevorzugt von 3,6 bis 4,5, aufweist,
und
B) mindestens ein hydroxy-alkylmodifziertes Guar,
**dadurch gekennzeichnet, dass** das hydroxy-alkylmodifzierte Guar mit HydroxyAlkylgruppen mit 12 bis 26 Kohlenstoffatomen modifiziert ist.

2. Zusammensetzung gemäß Anspruch 1, **dadurch gekennzeichnet, dass** der Polyglycerinester nach seiner vollständiger Hydrolyse im Mittel (Zahlenmittel) pro Mol Polyglycerinester
von 0,01 bis 0,07 Mol, bevorzugt von 0,01 bis 0,50 Mol, besonders bevorzugt von 0,01 bis 0,30 Mol, mindestens einer Carbonsäure a)
von 0,10 bis 1,70 Mol, bevorzugt von 0,30 bis 1,50 Mol, besonders bevorzugt von 0,40 bis 1,40 Mol, mindestens einer Carbonsäure b)
von 0,01 bis 0,80 Mol, bevorzugt von 0,01 bis 0,60 Mol, besonders bevorzugt von 0,05 bis 0,40 Mol, mindestens einer Carbonsäure c)

freisetzt.

3. Zusammensetzung gemäß Anspruch 1 oder 2, **dadurch gekennzeichnet, dass** das Molverhältnis der nach vollständiger Hydrolyse des Polyglycerinesters erhaltenen Carbonsäure a) zu Carbonsäure b) zu Carbonsäure c)
0,6 bis 1,4 : 16,5 bis 20,5 : 3,0 bis 4,8, bevorzugt
0,8 bis 1,2 : 17,5 bis 19,5 : 3,5 bis 4,3, besonders bevorzugt
0,9 bis 1,1 : 18,0 bis 19,0 : 3,7 bis 4,1,
beträgt.

4. Zusammensetzung gemäß mindestens einem der vorherigen Ansprüche, **dadurch gekennzeichnet, dass** die Carbonsäuren a), b) und c) ausgewählt sind aus linearen, gesättigten, unsubstituierten Carbonsäuren.

5. Zusammensetzung gemäß mindestens einem der vorherigen Ansprüche, **dadurch gekennzeichnet, dass** die Carbonsäure a) ausgewählt ist aus Caprylsäure und Caprinsäure, die Carbonsäure b) ausgewählt ist aus Stearinsäure und Palmitinsäure und die Carbonsäure c) Behensäure ist.

6. Zusammensetzung gemäß mindestens einem der vorherigen Ansprüche, **dadurch gekennzeichnet, dass** das nach vollständiger Hydrolyse des Polyglycerinesters freigesetzte Polyglycerin ein Massenverhältnis von Glycerin zu Diglycerin von größer 1, bevorzugt größer 1,2, besonders bevorzugt größer 1,4 aufweist.

7. Zusammensetzung gemäß mindestens einem der vorherigen Ansprüche, **dadurch gekennzeichnet, dass** das hydroxy-alkylmodifzierte Guar mit HydroxyAlkylgruppen mit 18 bis 24, bevorzugt 20 bis 22, Kohlenstoffatomen modifiziert ist.

8. Zusammensetzung gemäß mindestens einem der vorherigen Ansprüche, **dadurch gekennzeichnet, dass** das hydroxy-alkylmodifzierte Guar zusätzlich hydroxypropyl modifiziert ist.

9. Zusammensetzung gemäß mindestens einem der vorherigen Ansprüche, **dadurch gekennzeichnet, dass** sie zusätzlich enthält C) mindestens einen ausgewählt aus Deo-Wirkstoff und AP-Wirkstoff.

10. Zusammensetzung gemäß mindestens einem der vorherigen Ansprüche, **dadurch gekennzeichnet, dass** der AP-Wirkstoff ausgewählt ist aus der Gruppe umfassend, bevorzugt bestehend aus, Aluminium-Salzen und Zirkonium-Salzen sowie der Deo-Wirkstoff aus der Gruppe der Zink-Salze, insbesondere Aluminium-Chlorohydrat und Zinkricinoleate.

11. Zusammensetzung gemäß mindestens einem der vorherigen Ansprüche, **dadurch gekennzeichnet, dass** sie eine Emulsion, insbesondere eine Öl-in-Wasser-Emulsion ist.

12. Zusammensetzung gemäß mindestens einem der vorherigen Ansprüche, **dadurch gekennzeichnet, dass** sie eine Viskosität in einem Bereich von 500 bis 20000, bevorzugt von 1500 bis 10000 mPas aufweist.

13. Zusammensetzung gemäß mindestens einem der vorherigen Ansprüche, **dadurch gekennzeichnet, dass** sie polyglycoletherfrei und frei von alkoxylierten Verbindungen ist.

**Claims**

1. Composition comprising

   A) polyglycerol ester,
   which, after its complete hydrolysis, releases

   a) at least one carboxylic acid having 6 to 14, preferably 8 to 12, carbon atoms,
   b) at least one carboxylic acid having 16 to 20, preferably 18 to 20, carbon atoms,
   c) at least one carboxylic acid having 22 to 28, preferably 22 to 24, carbon atoms,
   and
   which, after its complete hydrolysis, releases a polyglycerol having an average degree of polymerization of from 3.0 to 5.0, preferably from 3.3 to 4.7, particularly preferably from 3.6 to 4.5,

and

B) at least one hydroxyalkyl-modified guar, **characterized in that** the hydroxyalkyl-modified guar has been modified with hydroxyalkyl groups having 12 to 26 carbon atoms.

2. Composition according to Claim 1, **characterized in that** the polyglycerol ester, after its complete hydrolysis, releases an average (number average) per mole of polyglycerol ester of
from 0.01 to 0.07 mol, preferably from 0.01 to 0.50 mol, particularly preferably from 0.01 to 0.30 mol, of at least one carboxylic acid a)
from 0.10 to 1.70 mol, preferably from 0.30 to 1.50 mol, particularly preferably from 0.40 to 1.40 mol, of at least one carboxylic acid b)
from 0.01 to 0.80 mol, preferably from 0.01 to 0.60 mol, particularly preferably from 0.05 to 0.40 mol, of at least one carboxylic acid c).

3. Composition according to Claim 1 or 2, **characterized in that** the molar ratio of carboxylic acid a) to carboxylic acid b) to carboxylic acid c) obtained after complete hydrolysis of the polyglycerol ester is
0.6 to 1.4:16.5 to 20.5:3.0 to 4.8, preferably
0.8 to 1.2:17.5 to 19.5:3.5 to 4.3, particularly preferably 0.9 to 1.1:18.0 to 19.0:3.7 to 4.1.

4. Composition according to at least one of the preceding claims, **characterized in that** the carboxylic acids a), b) and c) are selected from linear, saturated, unsubstituted carboxylic acids.

5. Composition according to at least one of the preceding claims, **characterized in that** carboxylic acid a) is selected from caprylic acid and capric acid, carboxylic acid b) is selected from stearic acid and palmitic acid and carboxylic acid c) is behenic acid.

6. Composition according to at least one of the preceding claims, **characterized in that** the polyglycerol released after complete hydrolysis of the polyglycerol ester has a mass ratio of glycerol to diglycerol of greater than 1, preferably greater than 1.2, particularly preferably greater than 1.4.

7. Composition according to at least one of the preceding claims, **characterized in that** the hydroxyalkyl-modified guar is modified with hydroxyalkyl groups having 18 to 24, preferably 20 to 22, carbon atoms.

8. Composition according to at least one of the preceding claims, **characterized in that** the hydroxyalkyl-modified guar has additionally been hydroxypropyl-modified.

9. Composition according to at least one of the preceding claims, **characterized in that** said composition additionally comprises
C) at least one selected from deodorant active ingredient and antiperspirant active ingredient.

10. Composition according to at least one of the preceding claims, **characterized in that** the antiperspirant active ingredient is selected from the group comprising, preferably consisting of, aluminium salts and zirconium salts, and the deodorant active ingredient from the group of zinc salts, in particular aluminium chlorohydrate and zinc ricinoleate.

11. Composition according to at least one of the preceding claims, **characterized in that** said composition is an emulsion, particularly an oil-in-water emulsion.

12. Composition according to at least one of the preceding claims, **characterized in that** said composition has a viscosity in a range from 500 to 20 000, preferably 1500 to 10 000 mPas.

13. Composition according to at least one of the preceding claims, **characterized in that** said composition is free from polyglycol ethers and free from alkoxylated compounds.


**Revendications**

1. Composition contenant :

A) un ester de polyglycérine,
qui libère après son hydrolyse totale

    a) au moins un acide carboxylique contenant 6 à 14, de préférence 8 à 12, atomes de carbone,
    b) au moins un acide carboxylique contenant 16 à 20, de préférence 18 à 20, atomes de carbone,
    c) au moins un acide carboxylique contenant 22 à 28, de préférence 22 à 24, atomes de carbone,

et
qui libère après son hydrolyse totale une polyglycérine qui présente un degré de polymérisation moyen de 3,0 à 5,0, de préférence de 3,3 à 4,7, de manière particulièrement préférée de 3,6 à 4,5,
et
B) au moins une gomme de guar à modification hydroxyalkyle,
**caractérisée en ce que** la gomme de guar à modification hydroxyalkyle est modifiée avec des groupes hydroxyalkyle contenant 12 à 26 atomes de carbone.

2. Composition selon la revendication 1, **caractérisée en ce que** l'ester de polyglycérine libère après son hydrolyse totale en moyenne (moyenne en nombre) par mole d'ester de polyglycérine
de 0,01 à 0,07 mole, de préférence de 0,01 à 0,50 mole, de manière particulièrement préférée de 0,01 à 0,30 mole, d'au moins un acide carboxylique a),
de 0,10 à 1,70 mole, de préférence de 0,30 à 1,50 mole, de manière particulièrement préférée de 0,40 à 1,40 mole, d'au moins un acide carboxylique b),
de 0,01 à 0,80 mole, de préférence de 0,01 à 0,60 mole, de manière particulièrement préférée de 0,05 à 0,40 mole, d'au moins un acide carboxylique c).

3. Composition selon la revendication 1 ou 2, **caractérisée en ce que** le rapport molaire entre l'acide carboxylique a) et l'acide carboxylique b) et l'acide carboxylique c) obtenus après l'hydrolyse totale de l'ester de polyglycérine est de 0,6 à 1,4:16,5 à 20,5:3,0 à 4,8, de préférence de 0,8 à 1,2:17,5 à 19,5:3,5 à 4,3, de manière particulièrement préférée de 0,9 à 1,1:18,0 à 19,0:3,7 à 4,1.

4. Composition selon au moins l'une quelconque des revendications précédentes, **caractérisée en ce que** les acides carboxyliques a), b) et c) sont choisis parmi les acides carboxyliques linéaires, saturés, non substitués.

5. Composition selon au moins l'une quelconque des revendications précédentes, **caractérisée en ce que** l'acide carboxylique a) est choisi parmi l'acide caprylique et l'acide caprique, l'acide carboxylique b) est choisi parmi l'acide stéarique et l'acide palmitique, et l'acide carboxylique c) est l'acide béhénique.

6. Composition selon au moins l'une quelconque des revendications précédentes, **caractérisée en ce que** la polyglycérine libérée après l'hydrolyse totale de l'ester de polyglycérine présente un rapport en masse entre la glycérine et la diglycérine supérieur à 1, de préférence supérieur à 1,2, de manière particulièrement préférée supérieur à 1,4.

7. Composition selon au moins l'une quelconque des revendications précédentes, **caractérisée en ce que** la gomme de guar à modification hydroxyalkyle est modifiée avec des groupes hydroxyalkyle contenant 18 à 24, de préférence 20 à 22, atomes de carbone.

8. Composition selon au moins l'une quelconque des revendications précédentes, **caractérisée en ce que** la gomme de guar à modification hydroxyalkyle est en outre modifiée par des groupes hydroxypropyle.

9. Composition selon au moins l'une quelconque des revendications précédentes, **caractérisée en ce qu'**elle contient en outre :
C) au moins un choisi parmi un agent actif déo et un agent actif AP.

10. Composition selon au moins l'une quelconque des revendications précédentes, **caractérisée en ce que** l'agent actif AP est choisi dans le groupe comprenant, de préférence constitué par, les sels d'aluminium et les sels de zirconium, et l'agent actif déo est choisi dans le groupe des sels de zinc, notamment le chlorohydrate d'aluminium et le ricinoléate de zinc.

11. Composition selon au moins l'une quelconque des revendications précédentes, **caractérisée en ce qu'**il s'agit d'une émulsion, notamment d'une émulsion huile dans eau.

**12.** Composition selon au moins l'une quelconque des revendications précédentes, **caractérisée en ce qu'**elle présente une viscosité dans une plage allant de 500 à 20 000, de préférence de 1 500 à 10 000 mPas.

**13.** Composition selon au moins l'une quelconque des revendications précédentes, **caractérisée en ce qu'**elle est exempte d'éther de polyglycol et est exempte de composés alcoxylés.

Abbildung 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013092186 A
- WO 2015132053 A
- US 4960876 A
- US 20070071701 A
- US 3792068 A
- US 2854382 A
- EP 2421614 A
- DE 10361202
- EP 2705832 A